# EUROPEAN PATENT SPECIFICATION

(11) **EP 1 605 890 B1**
(45) Date of publication and mention of the grant of the patent: **28.09.2011**
(21) Application number: 04758545.0
(22) Date of filing: 26.03.2004
(51) Int. Cl.: A61J 1/03

(54) **KIT FOR PHARMACEUTICAL USE**
KIT ZUR PHARMAZEUTISCHEN VERWENDUNG
KIT UTILISABLE A DES FINS PHARMACEUTIQUES

(30) Priority: 26.03.2003 US 457865 P
(43) Date of publication of application: 21.12.2005
(73) Proprietor: Warner Chilcott Company, LLC, Puerto Rico 00738-1005 (PR)
(72) Inventor: CAWTHRAY, Richard, James, F-74160 Collonges-sous-Saleve (FR); DIFABRITUS, Vincent, Anthony, Mason, OH 45040 (US); LOUGHREN, Ellen, Mary, Oxford, NY 13830 (US); TROMBLEY, Kurt, Franklin, Loveland, OH 45140 (US); VAN DER GEEST, Stephanus, Alexander, Paulus, F-01220 Divonne les Bains (FR)
(74) Representative: O'Connell, Maura
(86) International application number: PCT/US2004/009595
(87) International publication number: WO 2004/087038

(56) References cited:
- US-A- 4 736 849
- US-A1- 2001 044 427
- US-A1- 2002 066 691

## Description

### FIELD OF THE INVENTION

The present invention relates to kits for the pharmaceutical administration of an bisphosphonate ingredient and one or more accompanying calcium-containing nutrients. These kits are particularly useful for treatment regimens wherein the bisphosphonate is administered on a continuous frequency other than daily and the calcium-containing nutrient is administered on the days in between the days the active ingredient is administered.

### BACKGROUND OF THE INVENTION

With many treatment regimens, to achieve maximum efficiency of the active ingredients it is advisable to supplement the regimen with one or more nutrients. Therefore, the patient must remember not only to take the active ingredient, but also the associated nutrient. These dosages may require administration at different times of the day or under different conditions, for example, on an empty stomach vs. a full stomach. In addition, when the pharmaceutical active is not administered every day, remembering which day the active is to be taken can be confusing to the patient. Patient compliance with these types of programs is therefore an issue.

Many types of kits have been developed for dispensing pharmaceutical actives. Such kits include those designed to dispense active ingredients on a continuous daily frequency. See, e.g., U.S. Pat. No. 5,265,728, to Allendorf et al., issued Nov. 30, 1993; EP Pub. 0 511 726 A2, to Berlex Laboratories, Inc., published Nov. 4, 1992; PCT Pub. WO 99/51214, to Akzo Nobel, published Oct. 14, 1999; and U.S. Pat No. 4,958,736, to Urheim, issued Sept 25, 1990, which describe dispensers for administering various pharmaceuticals, including oral contraceptives, on a continuous daily basis, including regimens wherein the active ingredient is administered daily for about 21 days followed by placebo administration for about seven days. Other kits and dispensers have been developed that are designed for administering multiple doses of the same active ingredient per day, or for the concurrent or nonconcurrent administration of two or more active agents. See, e.g., U.S. Pat No. 6,024,222, to Friberg et al., issued Feb. 15, 2000; U.S. Pat. No. 6,219,997, to Friberg et al., issued Apr. 24, 2001; U.S. Pat Pub. 2003/0168376 Al, Taneja et al., published Sept. 11, 2003; U.S. Pat. Pub. 2003/0111479, Taneja et al., published June 19, 2003; U.S. Pat. No. 6,375,956, to Hermelin et al., issued Apr. 23, 2002; PCT Pub. WO 88/02342, Astra Likemedel Aktiebolag, published Apr. 7, 1988; U.S. Pat. No. 4,295,567, to Knudsen, issued Oct 20, 1981; DE 297 19 070, to Byk Gulden Lomberg Chemische Fabrik, published June 25, 1998; U.S. Pat No. 5,848,976, to Weinstein, issued Dec. 15, 1998; U.S. Pat No. 6,270,796, to Weinstein, issued Aug. 7, 2001; U.S. Pat. No. 6,564,945, to Weinstein et al., issued May 20, 2003; and U.S. Pat. No. 5,788,974, to D'Amico et al., issued Aug. 4, 1998. A kit has also been disclosed for the administration of an active ingredient on a once weekly basis. See U.S. Pub. 2001/0044427, Mazel et al., published Nov. 22, 2001. However, none of these aforementioned kits or dispensers is designed or intended to address the compliance issues associated with the continuous administration of a pharmaceutical active on a frequency other than daily together with taking a separate associated nutrient on the days in between the days the active ingredient is administered.

Applicants have developed a dispensing means that addresses the issues presented. Applicants have found that the present invention simplifies complex therapies and aids patients in understanding how to take their medication and accompanying nutrients, which can then lead to greater compliance with complicated treatment regimens, for example, regimens wherein the patient takes a unit dose of an active ingredient on a continuous frequency other than daily and a unit dose of a nutrient on the days in between the days the patient takes the active dose.

The present invention is useful in treatment regimens wherein the active ingredient is a bisphosphonate and the nutrient is calcium or a calcium-containing supplement. Patients taking bisphosphonates are generally instructed to take a daily calcium supplement, however, the bisphosphonate and the calcium supplement should not be taken at the same time. Because bisphosphonates chelate calcium, taking a unit dose of a bisphosphonate at the same time as a calcium supplement interferes with the absorption of the bisphosphonate, thereby potentially decreasing the efficacy of the bisphosphonate. The kit of the present invention addresses this issue. By taking the unit doses of nutrient on the days in between the days the patient takes the active dose, the patient avoids the problems associated with the simultaneous dosing of both a bisphosphonate and a calcium-containing supplement. Applicants have found that the present invention aids patients in understanding when and how to take bisphosphonates and calcium-containing supplements, which can lead to greater patient compliance and maximum benefit from such treatment regimens.

### SUMMARY OF THE INVENTION

The present invention relates to a kit (i.e., article of manufacture) according to claim 1 for promoting the proper sequential oral administration of a bisphosphonate and accompanying calcium-containing nutrients, said kit comprising:
(a) at least one unit dose of a bisphosphonate to be given continuously on a frequency of once a week ;
(b) at least one unit dose of a nutrient to be given subsequent to the active dose administration and on the days in between the days when the unit dose of the bisphosphonate is taken; and
(c) a blister card individually and releasably containing the unit doses;
wherein said unit doses of bisphosphonate and nutrient are arranged horizontally or vertically in order of their use across the blister card.

The pharmaceutical active is a bisphosphonate and the nutrient is calcium, calcium and vitamin D, or a combined unit dose of calcium and vitamin D.

### BRIEF DESCRIPTION OF THE DRAWINGS

FIG. 1 is a plan view of the front of a blister card.
FIG. 2 is a plan view of the back of the blister card of FIG. 1.
FIG. 3 is a plan view of the front of a blister card.
FIG. 4 is a plan view of the front of a blister card.
FIG. 5 is a plan view of the front of a blister card.

### DETAILED DESCRIPTION OF THE INVENTION

The present invention relates to kits useful in treatment regimens comprising the administration of a bisphosphonate once a week and calcium and/or vitamin D on the days in between the days the patient takes the bisphosphonate dose.

The terms "bisphosphonate" and "diphosphonate," as used herein, include acids, salts, esters, and derivatives thereof. The bisphosphonates of the present invention include those preferred compounds containing a nitrogen atom. Nonlimiting examples of bisphosphonates useful herein include the following: 1-hydroxy-2-(3-pyridinyl)-ethylidene-1,1-bisphosphonic acid (risedronate) as described in U.S. Pat. No. 5,583,122, to Benedict et al., issued Dec. 10, 1996; 4-amino-1-hydroxybutylidene-1,1-bisphosphonic acid (alendronic acid or alendronate) as described in U.S. Pat. No. 4,621,077, to Rosini et al., issued Nov. 4, 1986; U.S. Pat. No. 4,922,007, to Kieczykowski et al., issued May 1, 1990; U.S. Pat. No. 5,019,651, to Kioczykowsld, issued May 28, 1991; 3-amino-1-hydroxypropylidene-1,1-bisphosphonic acid (pamidronate); (4-chlorophenyl)thiomethane-1,1-diphosphonic acid (tiludronate) as described in U.S. Pat. No. 4,876,248 to Breliere et al., issued Oct. 24, 1989; 1.1-dichloromethylene-1,1-diphosphonic acid (clodronate) as described in Belgium Patent 672,205 (1966); cycloheptylaminomethylene-1,1-bisphosphonic acid (cimadronate), as described in U.S. Pat. No. 4,970,335, to Isomura et aL, issued Nov. 13, 1990; 1-hydroxy-3-(N-methyl-N-pentylamino)propylidene-1,1-bisphosphonic acid (ibandronate), which is described in U.S. Pat. No. 4,927,814, issued May 22, 1990; 1-hydroxy-2-(imidazol-1-yl)ethane-1,1-bisphosphonic acid (zoledronate); and 1-(N-phenylaminothiocarbonyl)methane-1,1-bisphosphonic acid.

"Blister cards" are well known in the packaging industry and are widely used for packaging pharmaceutical unit doses. The blister cards of the present invention individually and releasably contain the unit doses of bisphosphonate and calcium-containing nutrient.

The terms "continuous" and "continuously," as used herein, mean at regular specified intervals. For example, a continuous frequency of once a week means that the active is given once a week for an unspecified period of time or for as long as treatment is necessary.

The term "once a week" or "once weekly" means that a unit dose is administered once a week, i.e., one time during a seven day period.

The term "unit dose" or unit dosage" means a dosage form containing an amount of bisphosphonate or calcium-containing nutrient suitable for administration in one single dose, according to sound medical practice. The kits of the present invention are particularly useful for the administration of unit doses in the form of tablets and capsules.

The term "combined unit dose of calcium and vitamin D," as used herein, means a single unit dose form comprising both calcium and vitamin D.

The term "IU," as used herein, means International Units. One microgram of vitamin D is approximately 40 International Units.

The treatment is the once weekly dosing of a bisphosphonate for the treatment of osteoporosis. The bisphosphonate is administered in an amount that has been determined to be therapeutically effective. For example, risedronate may be administered at a dose of 35mg per week. Alendronate may be administered at doses of 70 mg and/or 35 mg per week. Other treatment regimens for bisphosphonates may include those for cancer, Paget's disease, and other bone resorptive disorders.

The term "nutrient," as used herein, means any nutritional or dietary supplement including but not limited to vitamins, minerals, amino acids, herbs or other botanicals, or concentrates, metabolites, constituents, extracts, or combinations of the same.

The nutrients to be administered in the bisphosphonate regimen are calcium, calcium and vitamin D, or a combined unit dose of calcium and vitamin D. Oral forms of calcium suitable for use in the present invention include capsules, compressed tablets, chewable tablets, and the like. Typical salt forms of calcium suitable for use in the present invention include but are not limited to calcium carbonate, calcium citrate, calcium malate, calcium citrate malate, calcium glubionate, calcium gluceptate, calcium gluconate, calcium lactate, dibasic calcium phosphate, and tribasic calcium phosphate. Different salt forms of calcium contain different percentages of elemental calcium. For example, calcium carbonate contains about 40% elemental calcium (i.e., 1000 mg calcium carbonate contains about 400 mg elemental calcium). In one embodiment of the invention, calcium can be administered at doses of 400 mg to 1500 mg of elemental calcium per day, on the days in between the days when the patient takes a unit dose of pharmaceutical active.

The term "vitamin D," as used herein, refers to any form of vitamin D that may be administered to a mammal as a nutrient. Vitamin D is metabolized in the body to provide what is often referred to as "activated" forms of vitamin D. The term "vitamin D" can include activated and non-activated forms of vitamin D, as well as precursors and metabolites of such forms. Precursors of these activated forms include vitamin D₂ (ergocalciferol, produced in plants) and vitamin D₃ (cholecalciferol, produced in skin and found in animal sources and used to fortify foods). Vitamins D₂ and D₃ have similar biological efficacy in humans. Non-activated metabolites of vitamins D₂ and D₃ include hydroxylated forms of vitamins D₂ and D₃. Activated vitamin D analogs cannot be administered in large doses on an intermittent schedule, due to their toxicity in mammals. However, non-activated vitamin D₂, vitamin D₃, and their metabolites may be administered in larger doses than "active" forms of vitamin D on an intermittent basis, without toxicity.

In another embodiment of the invention, the nutrient is a unit dose comprising both calcium and vitamin D. In a further embodiment, the unit dose comprises 600 mg elemental calcium and 400 IU vitamin D, to be administered on the days in between the days when the patient takes the unit dose of the pharmaceutical active.

The kits of the present invention are useful for administering bisphosphonates on a weekly basis. The kits comprise at least one unit dose of a pharmaceutical active and at least one unit dose of a nutrient on a single blister card. In addition, the kits may include means for aiding the memory on the card. More than one week of dosage units may be present on one card and more than one card may be packaged together.

In one embodiment of the present invention, the blister card comprises a means for aiding the memory. Means for aiding the memory can include but are not limited to a listing of the days of the week, numbering, illustrations, arrows, Braille, calendar stickers, reminder cards, or other means specifically selected by the patient.

The kit of the present invention provides a blister card comprising a unit dose of a pharmaceutical active suitable for once weekly dosing, and six unit doses of a nutrient, to be taken on the days in between the days the patient takes the weekly pharmaceutical active unit dose. In another embodiment of the invention, the blister card comprises a unit dose of a pharmaceutical active suitable for once weekly dosing and twelve unit doses of a nutrient, wherein two doses of nutrient are taken each day on the days in between the days the patient takes the weekly pharmaceutical active. The pharmaceutical active is a bisphosphonate and the nutrient is calcium, calcium and vitamin D, or a combined unit dose of calcium and vitamin D.

In a further embodiment of the present invention, the blister card comprises, in vertical arrangement in order of their use, a unit dose of a pharmaceutical active followed by six unit doses of a nutrient. In another embodiment of the invention, the blister card comprises, in vertical arrangement in order of their use, a unit dose of a pharmaceutical active suitable for once weekly dosing followed by twelve unit doses of a nutrient, wherein two doses of nutrient are taken each day on the days in between the days the patient takes the weekly pharmaceutical active. When two unit doses of nutrient are to be taken on the same day, the daily allotment of unit doses of nutrient can be arranged vertically or horizontally across the blister card. In a further embodiment of the invention, the kit can comprise at least two blister cards, wherein each blister card comprises doses suitable for one week of treatment therapy. In another embodiment, the kit can comprise at least four blister cards, wherein each blister card comprises doses suitable for one week of treatment therapy. In another embodiment of the invention, the blister card comprises two vertical columns of unit doses, wherein each vertical column comprises one unit dose of a pharmaceutical active and six or twelve unit doses of a nutrient. In a further embodiment of the invention, the blister card comprises four vertical columns of unit doses, wherein each vertical column comprises one unit dose of a pharmaceutical active and six or twelve unit doses of a nutrient. In yet another embodiment of the present invention, the blister card comprises two vertical columns of doses, wherein each column comprises unit doses sufficient for two weeks of therapy, arranged in order of their use (i.e., two vertical columns, each comprising a first unit dose of a pharmaceutical active, followed by six unit doses of a nutrient, followed by a second unit dose of a pharmaceutical active, followed by six more unit doses of a nutrient, for a total of fourteen unit doses per column). The pharmaceutical active is a bisphosphonate and the nutrient is calcium, calcium and vitamin D, or a combined unit dose of calcium and vitamin D.

In an alternate embodiment of the invention, the blister card comprises, in horizontal arrangement in order of their use, a unit dose of a pharmaceutical active followed by six unit doses of a nutrient. In another embodiment of the invention, the blister card comprises, in horizontal arrangement in order of their use, a unit dose of a pharmaceutical active suitable for once weekly dosing followed by twelve unit doses of a nutrient, wherein two doses of nutrient are taken each day on the days in between the days the patient takes the weekly pharmaceutical active. When two unit doses of nutrient are to be taken on the same day, the daily allotment of unit doses of nutrient can be arranged vertically or horizontally across the blister card. In a further embodiment of the invention, the kit can comprise at least two blister cards, wherein each blister card comprises doses suitable for one week of treatment therapy. In another embodiment, the kit can comprise at least four blister cards, wherein each blister card comprises doses suitable for one week of treatment therapy. In another embodiment of the invention, the blister card comprises two horizontal rows of unit doses, wherein each horizontal row comprises one unit dose of a pharmaceutical active and six or twelve unit doses of a nutrient. In a further embodiment of the invention, the blister card comprises four horizontal rows of unit doses, wherein each horizontal row comprises one unit dose of a pharmaceutical active and six or twelve unit doses of a nutrient. In yet another embodiment of the present invention, the blister card comprises two horizontal rows of doses, wherein each row comprises unit doses sufficient for two weeks of therapy, arranged in order of their use (i.e., two horizontal rows, each comprising a first unit dose of a pharmaceutical active, followed by six unit doses of a nutrient, followed by a second unit dose of a pharmaceutical active, followed by six more unit doses of a nutrient, for a total of fourteen unit doses per row). The pharmaceutical active is a bisphosphonate and the nutrient is calcium, calcium and vitamin D, or a combined unit dose of calcium and vitamin D.

The present invention provides a kit for providing complex therapeutic regimens to patients in a simplified manner, which can then lead to increased patient compliance. The figures exemplify an embodiment of the present invention.

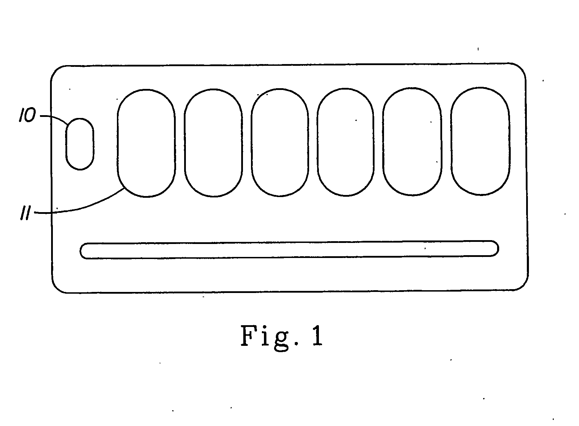

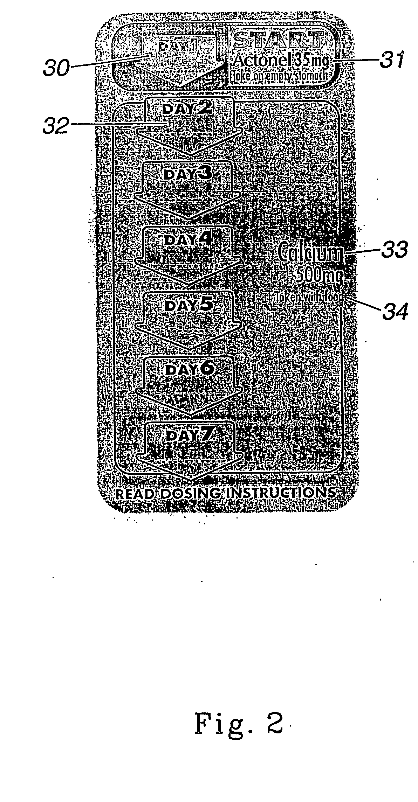

Referring to FIG. 1, the blister card comprises cavities 10 in which the unit doses of the pharmaceutical active are contained. The general structure of these blister cards is well known in the art. These can comprise a clear or opaque film layer containing blister cavities 10 heat-sealed to a foil layer which includes indicia on one or both sides. The blister card further comprises cavities 11 in which the unit doses of nutrient are contained. It is appreciated that the individual blisters may vary in size and shape, depending on the size of shape of the unit dose of pharmaceutical active or nutrient releasably contained therein. As illustrated in FIG. 2, each blister card is printed with information to aid the patient in taking the doses. Such information includes the relative order of use in the treatments 30, the product name 31 and 33, and instructions as to when or how to take the dose 34.

The blister card of one embodiment of the present invention, presented in FIG. 1, contains one cavity 10 in which the unit dose of a pharmaceutical active is contained and six cavities 11 in which the unit doses of nutrient are contained to be taken on subsequent days for one week. The back of the blister card, FIG.2, provides memory aids 32 to reflect the appropriate information for proper dosing.

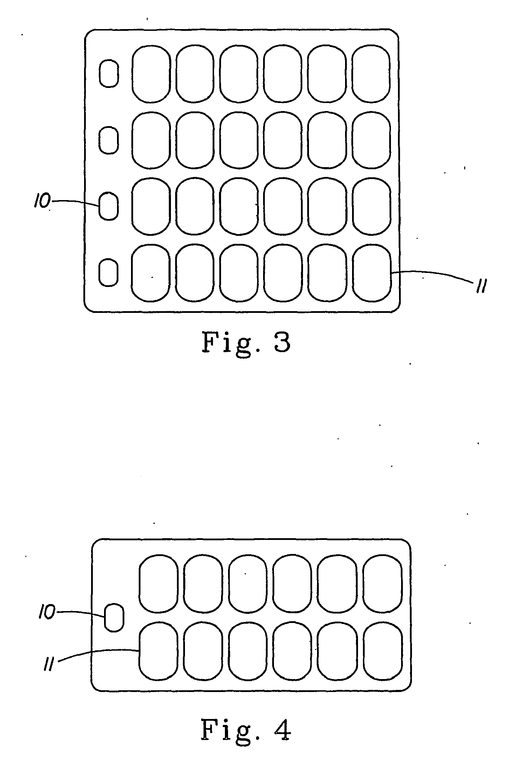

Referring to FIG. 3, another embodiment of the invention is shown wherein the blister card comprises four rows, each row containing one cavity 10 in which the unit dose of the pharmaceutical active is contained and six cavities 11 in which the unit doses of nutrient are contained. The patient takes one unit dose of a pharmaceutical active and then takes six unit doses of a nutrient on subsequent days, repeating this process four times.

Referring to FIG. 4, yet another embodiment of the invention is shown wherein the blister card comprises one cavity 10 in which the unit dose of the pharmaceutical active is contained and twelve cavities 11 in which the unit doses of nutrient are contained. The patient takes one unit dose of a pharmaceutical active and then takes two unit doses of a nutrient each day on subsequent days for one week.

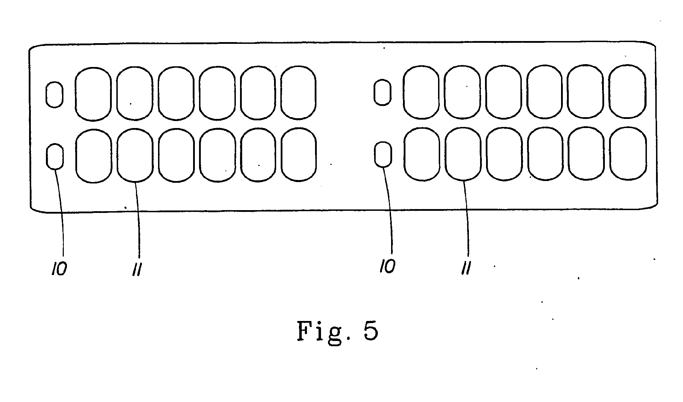

Referring to FIG. 5, still another embodiment of the invention is shown wherein the blister card comprises two rows, each row containing a first cavity 10 in which a unit dose of pharmaceutical active is contained, followed by six cavities 11 in which unit doses of nutrient are contained, followed by a second cavity 10 in which a unit dose of pharmaceutical active is contained, followed by six more cavities 11 in which unit doses of nutrient are contained. In this embodiment, each row contains unit doses of pharmaceutical active and nutrient sufficient for two weeks of therapy when the pharmaceutical active is taken on a once weekly basis.

### EXAMPLES

### Example 1

A 75-year-old female patient diagnosed with osteoporosis is prescribed a weekly dose of 35 mg risedronate, in combination with calcium. The patient has difficulty remembering which day of the week she takes the risedronate dose, and occasionally forgets to take the calcium supplement, or takes the calcium supplement at the same time as she takes the risedronate weekly dose, thereby reducing the efficacy of the risedronate unit dose. The patient is presented with a blister card of the present invention, which contains in horizontal arrangement a unit dose of risedronate followed by six unit doses of 600 mg each elemental calcium. The blister card contains printed information which instructs the patient as to how and in which order the doses are to be taken. The patient finds that this blister card is easy to use and aids her in remembering to take the risedronate on a weekly basis as well as the calcium supplement on the days in between the days she takes the risedronate doses. The patient also avoids taking a calcium supplement at the same time as she takes the risedronate unit dose, thus avoiding undesired interaction between the two. The patient shows increased compliance with her prescribed treatment regimen.

### Example 2

A 55-year-old female patient at risk for osteoporosis is prescribed a weekly dose of 35 mg risedronate in combination with a calcium and vitamin D supplement, as a preventative measure. The patient is presented with a blister card of the present invention, which contains four rows in horizontal arrangement, each row containing a unit dose of risedronate followed by six unit doses of a nutrient, each containing 600 mg elemental calcium and 400 IU vitamin D. The blister card contains printed information which instructs the patient as to how and in which order the doses are to be taken. The patient uses this blister card for four weeks and finds that the package aids her in understanding her therapy and complying with her doctor's prescribed treatment regimen. The patient complies with instructions to take risedronate once a week and to take a supplement of calcium and vitamin D on the days in between the days she takes the risedronate doses, in accordance with her doctor's instructions.

### Example 3

A 67-year-old female patient diagnosed with osteoporosis is prescribed a weekly dose of 70 mg alendronate, in combination with calcium. The patient has difficulty remembering which day of the week she takes the alendronate dose, and frequently forgets to take the calcium supplement. The patient is presented with a blister card of the present invention, which contains in vertical arrangement a unit dose of alendronate followed by six unit doses of 600 mg each elemental calcium. The blister card contains printed information which instructs the patient as to how and in which order the doses are to be taken. The patient finds that this blister card is easy to use, and that it aids her in remembering to take the alendronate on a weekly basis as well as the calcium supplement on the days in between the days she takes the alendronate doses. The patient shows increased compliance with her prescribed treatment regimen.

### Example 4

A 58-year-old female patient at risk for osteoporosis is prescribed a weekly dose of 35 mg alendronate in combination with a calcium and vitamin D supplement, as a preventative measure. The patient is presented with a blister card of the present invention, which contains four rows in vertical arrangement, each row containing a unit dose of alendronate followed by six unit doses of a nutrient, each containing 600 mg elemental calcium and 400 IU vitamin D. The blister card contains printed information which instructs the patient as to how and in which order the doses are to be taken. The patient uses this blister card for four weeks and finds that the package aids her in understanding her therapy and complying with her doctor's prescribed treatment regimen. The patient complies with instructions to take alendronate once a week, and to take a supplement of calcium and vitamin D on the days in between the days she takes the alendronate doses.

### Example 5

A 72-year-old female patient diagnosed with osteoporosis is prescribed a weekly dose of 35 mg risedronate, in combination with 1200 mg elemental calcium daily, divided into two unit doses each day. The patient has difficulty remembering which day of the week she takes the risedronate dose, and occasionally forgets to take the calcium supplements. The patient is presented with a blister card of the present invention, which contains in horizontal arrangement a unit dose of risedronate followed by twelve unit doses of 600 mg each elemental calcium. The twelve unit doses of calcium are arranged in two rows following the unit dose of risedronate, as pictured in FIG. 4. The blister card contains printed information which instructs the patient as to how and in which order the doses are to be taken. The patient finds that this blister card is easy to use and aids her in understanding her therapy and remembering to take the risedronate on a weekly basis as well as the calcium supplements on the days in between the days she takes the risedronate doses. The patient is able to understand her treatment regimen, and shows increased compliance therewith.

### Example 6

A 65-year-old female patient diagnosed with osteoporosis is prescribed a weekly dose of 35 mg risedronate in combination with a calcium and vitamin D supplement. The patient is presented with a blister card of the present invention, which contains two rows of unit doses in horizontal arrangement, each row containing a unit dose of risedronate followed by six unit doses of a nutrient, followed by a second unit dose of risedronate, followed by six more unit doses of nutrient, as pictured in FIG. 5. Each unit dose of nutrient contains 600 mg elemental calcium and 400 IU vitamin D. The blister card contains printed information which instructs the patient as to how and in which order the doses are to be taken. The patient uses this blister card for four weeks and finds that the package aids her in understanding her therapy and complying with her doctor's prescribed treatment regimen. The patient complies with instructions to take risedronate once a week and to take a supplement of calcium and vitamin D on the days in between the days she takes the risedronate doses, in accordance with her doctor's instructions.

### Example 7

A 65-year-old female patient diagnosed with osteoporosis is currently taking risedronate on a weekly basis in combination with a calcium supplement. The patient is first shown a blister card of the present invention, which contains in vertical arrangement a unit dose of risedronate, followed by six unit doses of calcium. The blister card contains printed information which instructs the patient as to how and in which order the doses are to be taken. The patient is then shown a second blister card having an alternate horizontal arrangement of unit doses, wherein the first row contains one unit dose of risedronate located in the center of the card, and the second row contains a horizontal arrangement of six unit doses of calcium. The second blister card also contains printed information which instructs the patient as to how and in which order the doses are to be taken. The patient prefers the first blister card over the second blister card. She determines that the arrangement of unit doses of the first blister card would aid her in remembering to take both the risedronate active and the calcium supplements on the correct days and in the correct manner. She also determines that the arrangement of the doses on the first blister card is clear and less confusing than the arrangement of the unit doses on the second blister card.

### Example 8

A 70-year-old female patient diagnosed with osteoporosis is currently taking risedronate on a weekly basis in combination with a calcium supplement. The patient is first shown a blister card of the present invention, which contains in horizontal arrangement a unit dose of risedronate, followed by six unit doses of calcium. The blister card contains printed information which instructs the patient as to how and in which order the doses are to be taken. The patient is then shown a second blister card having an alternate horizontal arrangement of unit doses, wherein the first row contains one unit dose of risedronate located at the left-hand side of the card, and the second row contains a horizontal arrangement of seven unit doses of calcium. The second blister card also contains printed information which instructs the patient as to how and in which order the doses are to be taken. The patient prefers the first blister card over the second blister card. She determines that the arrangement of unit doses of the first blister card would aid her in remembering to take both the risedronate active and the calcium supplements on the correct days and in the correct manner. She also determines that the arrangement of the doses on the first blister card is clear and less confusing than the arrangement of the unit doses on the second blister card.

## Claims

1. A kit for promoting the proper sequential oral administration of bisphosphonate and of accompanying nutrients on the days in between the days when the unit dose of the bisphosphonate is taken, said kit **characterized by**:
a) at least one unit dose of a bisphosphonate to be given continuously on a frequency of once a week;
b) at least one unit dose of a nutrient selected from calcium, calcium and vitamin D, and a combined unit dose of calcium and vitamin D to be given subsequent to the bisphosphonate administration and on the days in between the days when the unit dose of the bisphosphonate is taken; and
c) a blister card individually and releasably containing the unit doses;
wherein the blister card is **characterized by** one, two or four horizontal or vertical rows of unit doses, wherein each row is **characterized by** one unit dose of the bisphosphonate and six or twelve unit doses of the nutrient.

2. The kit of Claim 1 wherein the bisphosphonate is selected from the group consisting of risedronate, alendronate, pamidronate, tiludronate, cimadronate, ibandronate, and zoledronate.

3. The kit of Claim 1 wherein the kit is **characterized by** from two to four blister cards.

4. The kit of Claim 1 wherein the nutrient is calcium that is administered at doses of 400 mg to 1500 mg of elemental calcium per day, on the days in between the days when the unit dose of the bisphosphonate is taken.

5. The kit of Claim 1 wherein the nutrient is a unit dose comprising both calcium and vitamin D, the unit dose comprising 600 mg elemental calcium and 400 IU vitamin D, to be administered on the days in between the days when the unit dose of the bisphosphonate is taken.

6. The kit of any of the Claims 1-5 for use in increasing compliance with a treatment regimen.

## Patentansprüche

1. Kit zur Unterstützung der richtigen sequenziellen oralen Verabreichung von Bisphosphonat und von begleitenden Nährstoffen an den Tagen zwischen den Tagen, an denen die Einheitsdosis des Bisphosphonat eingenommen wird, wobei genannter Kit durch Folgendes gekennzeichnet ist:
a) mindestens eine Einheitsdosis eines Bisphosphonats, die kontinuierlich bei einer Häufigkeit von einmal pro Woche zu verabreichen ist;
b) mindestens eine Einheitsdosis eines Nährstoffs, der aus Calcium, Calcium und Vitamin D und einer kombinierten Einheitsdosis von Calcium und Viatmin D ausgewählt ist, die anschließend an die Verabreichung von Bisphosphonat und an den Tagen zwischen den Tagen, an denen die Einheitsdosis des Bisphosphonats eingenommen wird, zu verabreichen ist; und
c) eine Blisterkarte, die die Einheitsdosen einzeln und entnehmbar enthält;
worin die Blisterkarte durch eine, zwei oder vier horizontale oder vertikale Reihen an Einheitsdosen gekennzeichnet ist, worin jede Reihe durch eine Einheitsdosis des Bisphosphonats und sechs oder zwölf Einheitsdosen des Nährstoffs gekennzeichnet ist.

2. Kit nach Anspruch 1, worin das Bisphosphonat aus der Gruppe ausgewählt ist, die aus Risedronat, Alendronat, Pamidronat, Tiludronat, Cimadronat, Ibandronat und Zoledronat besteht.

3. Kit nach Anspruch 1, worin der Kit durch zwei bis vier Blisterkarten gekennzeichnet ist.

4. Kit nach Anspruch 1, worin der Nährstoff Calcium ist, das in Dosen von 400 mg bis 1500 mg von elementarem Calcium pro Tag an den Tagen zwischen den Tagen, an denen die Einheitsdosis des Bisphosphonats eingenommen wird, verabreicht wird.

5. Kit nach Anspruch 1, worin der Nährstoff eine Einheitsdosis ist, die sowohl Calcium als auch Viatmin D umfasst, wobei die Einheitsdosis 600 mg elementares Calcium und 400 IU Vitamin D umfasst, die an den Tagen zwischen den Tagen, an denen die Einheitsdosis des Bisphosphonats eingenommen wird, zu verabreichen ist.

6. Kit nach einem der Ansprüche 1-5 zur Verwendung bei der Steigerung der Compliance bezüglich eines Behandlungsregimes.

## Revendications

1. Kit destiné à favoriser l'administration orale séquentielle correcte de bisphosphonate et de nutriments annexes pour les jours entre les jours où la dose unitaire de bisphosphonate est prise, ledit kit étant **caractérisé par** :
a) au moins une dose unitaire d'un bisphosphonate à administrer en continu à une fréquence d'une fois par semaine ;
b) au moins une dose unitaire d'un nutriment choisi parmi le calcium, le calcium et la vitamine D, et une dose unitaire combinée de calcium et de vitamine D, à administrer suite à l'administration de bisphosphonate et les jours entre les jours où la dose unitaire de bisphosphonate est prise ; et
c) une carte à blisters contenant les doses unitaires de manière individuelle et libérable ;
où la carte à blisters est **caractérisée par** un, deux ou quatre rangs horizontaux ou verticaux de doses unitaires, où chaque rang est **caractérisé par** une dose unitaire de bisphosphonate et six ou douze doses unitaires de nutriment.

2. Kit selon la revendication 1, dans lequel le bisphosphonate est choisi dans le groupe constitué par le risédronate, l'alendronate, le pamidronate, le tiludronate, le cimadronate, l'ibandronate et le zolédronate.

3. Kit selon la revendication 1, dans lequel le kit est **caractérisé par** de deux à quatre cartes à blisters.

4. Kit selon la revendication 1, dans lequel le nutriment est le calcium, qui est administré à des doses allant de 400 mg à 1500 mg de calcium élémentaire par jour, les jours entre les jours où la dose unitaire de bisphosphonate est prise.

5. Kit selon la revendication 1, dans lequel le nutriment est une dose unitaire comprenant du calcium ainsi que de la vitamine D, la dose unitaire comprenant 600 mg de calcium élémentaire et 400 UI de vitamine D, à administrer les jours entre les jours où la dose unitaire de bisphosphonate est prise.

6. Kit selon l'une quelconque des revendications 1-5, pour une utilisation dans l'augmentation de l'observance vis-à-vis d'un régime de traitement.
